# EUROPÄISCHE PATENTSCHRIFT

(11) **EP 1 813 219 B1**
(45) Veröffentlichungstag und Bekanntmachung des Hinweises auf die Patenterteilung: **12.09.2012**
(21) Anmeldenummer: 07001255.4
(22) Anmeldetag: 20.01.2007
(51) Int. Cl.: A61B 19/00, F16M 11/04, A61G 13/12

(54) **Chirurgische Armstütze**
Surgical arm rest
Accoudoir chirurgical

(30) Priorität: 25.01.2006 DE 102006004126
(43) Veröffentlichungstag der Anmeldung: 01.08.2007
(73) Patentinhaber: Karl Storz GmbH & Co. KG, 78532 Tuttlingen (DE)
(72) Erfinder: Oberländer, Martin, 78234 Engen (DE); Sauer, Michael, 78532 Tuttlingen (DE)
(74) Vertreter: Heuckeroth, Volker

(56) Entgegenhaltungen:
- EP-A- 1 669 038
- WO-A-02/28338
- DE-A1- 3 032 415
- US-A- 5 074 501
- US-A- 5 791 263
- US-A- 5 918 330
- US-A1- 2005 051 688

## Beschreibung

Die Erfindung betrifft eine chirurgische Armstütze, mit einer Armauflage und einer Tragstruktur für die Armauflage, wobei die Tragstruktur eine Basis und zumindest einen Schwenkarm aufweist, der einerseits an der Basis um eine zumindest näherungsweise horizontale Schwenkachse verschwenkbar angelenkt und andererseits mit der Armauflage verbunden ist, so dass eine Verschwenkung des zumindest einen Schwenkarms um die Schwenkachse eine Höhenverstellung der Armauflage bewirkt, und wobei dem zumindest einen Schwenkarm ein Steuermechanismus zugeordnet ist, der wahlweise ein Feststellen und Verschwenken des zumindest einen Schwenkarms in Abwärtsrichtung und in Aufwärtsrichtung ermöglicht, wobei der Steuermechanismus eine Stange aufweist, die beim Verschwenken des zumindest einen Schwenkarms zumindest in ihrer Längsrichtung beweglich ist.

Eine Armstütze, die für chirurgische Zwecke verwendet werden kann, ist aus dem Dokument US 5,074,501 bekannt, gemäß dem Oberbegriff des Anspruchs 1.

Eine chirurgische Armstütze dient dazu, den Arm eines Chirurgen oder eines chirurgischen Assistenten während einer Operation zu stützen, um die Genauigkeit der Handbewegungen des Chirurgen zu erhöhen und Ermüdungen zu verringern. Berücksichtigt man, dass ein chirurgischer Eingriff mehrere Stunden dauern kann und das chirurgische Personal eine solche Operation in stehender Position ausführt, kann eine Armstütze dazu beitragen, dass die Genauigkeit der von der operierenden Person ausgeführten Handgriffe mit der Zeit nicht abnimmt.

Die aus dem oben genannten Dokument US 5,074, 501 bekannte Armstütze weist eine Armauflage auf, die an einer Tragstruktur für die Armauflage befestigt ist. Die Armauflage weist eine Basis und zwei Schwenkarme auf, die zusammen ein Parallelogramm ergeben. Die beiden Schwenkarme sind um jeweils eine Schwenkachse, die horizontal verläuft, an der Basis befestigt, wobei die beiden Schwenkachsen der beiden Schwenkarme in vertikaler Richtung voneinander beabstandet sind.

Die Armauflage ist mit den beiden Schwenkarmen an deren der Basis gegenüberliegenden Enden verbunden. Durch Verschwenken der Schwenkarme nach oben oder nach unten wird die Höhe der Armauflage verändert.

Den beiden Schwenkarmen der bekannten Armstütze ist ein Steuermechanismus zugeordnet, der es ermöglicht, die Schwenkarme nach oben oder nach unten zu verschwenken und somit die Höhe der Armauflage zu verändern und die Schwenkarme in einer bestimmten Schwenkstellung und damit die Armauflage in einer bestimmten Höhe festzustellen. Der Steuermechanismus weist eine Stange auf, die sich beim Verschwenken der beiden Schwenkarme zumindest in ihrer Längsrichtung bewegt.

Der Stange sind am oberen und unteren Schwenkarm Feststellklemmen zugeordnet, mit denen die Bewegung der Stange durch Festziehen der Klemmen blockiert werden kann, wodurch die Verschwenkung der beiden Schwenkarme blockiert wird und die Armauflage somit in einer bestimmten Höhe festgestellt werden kann.

Ein solcher Steuermechanismus zum Anheben bzw. Absenken und Feststellen der Armauflage hat den Nachteil, dass die Feststellklemmen nicht mit der Hand des Arms betätigt werden können, der sich gerade auf der Armauflage abstützt. Für chirurgische Anwendungen, bei denen mitunter die Höhe der Armauflage während einer Operation häufiger verstellt werden muss, ist diese bekannte Armstütze daher nur wenig geeignet, da sie umständlich zu handhaben ist. Außerdem ermöglicht sie kein kontrolliertes Absenken der Armauflage. Vielmehr besteht die Gefahr, dass nach dem Lösen der Feststellklemmen die Armauflage unkontrolliert absackt.

Dem gegenüber ist aus dem Dokument EP-A-1 486 178 eine chirurgische Armstütze bekannt, die gegenüber der vorbeschriebenen bekannten Armstütze den Vorteil hat, dass das Anheben, Absenken und Feststellen der Armauflage mit dem Arm durchgeführt werden kann, der in der Armauflage aufgenommen ist. Der Steuermechanismus dieser bekannten chirurgischen Armstütze weist einen Kraftlastschalter auf, der durch unterschiedliche Kraftausübung über den in der Armauflage aufgenommenen Arm einen Elektromotor ein- bzw. ausschaltet, wobei der Elektromotor eine Stange antreibt, die durch Ein- bzw. Ausfahren der Stange den Schwenkarm nach oben oder nach unten verschwenken lässt.

Der Nachteil eines solchen elektromechanischen Steuermechanismus besteht darin, dass eine Stromversorgung benötigt wird, die über ein Stromkabel erfolgt. Stromkabel stellen in einem Operationssaal jedoch Hindernisse dar. Darüber hinaus ist der Steuermechanismus bei einem Defekt des Elektromotors, der beispielsweise auf einer Überlastung des Elektromotors beruhen kann, funktionslos. Tritt ein solcher Defekt während einer Operation auf, muss die Operation ohne Armstütze fortgesetzt werden.

Für chirurgische Armstützen sind zum Einstellen der Höhe der Armauflage neben elektromotorischen Antrieben auch hydraulische oder pneumatische Steuermechanismen vorgeschlagen worden, wie beispielsweise in dem Dokument US 6,102,344. Ebenso wie elektromotorische Steuermechanismen haben hydraulische oder pneumatische Steuermechanismen den Nachteil, dass sie nicht für eine Sterilisation in einem Autoklaven geeignet sind. Chirurgische Instrumentarien und Apparaturen müssen besonders hohen Anforderungen an die Reinigbarkeit und insbesondere Sterilisierbarkeit erfüllen. Eine sichere Methode der Sterilisierung ist die Sterilisierung von Instrumenten und Apparaturen in einem Autoklaven, in dem Temperaturen von über 120°C und hohe Dampfdrücke herrschen. Diesen Bedingungen können weder elektromotorische noch hydraulische oder pneumatische Teile standhalten.

Eine chirurgische Armstütze, deren Steuermechanismus mechanisch arbeitet, ist daher wünschenswert.

Die aus dem eingangs genannten Dokument bekannte Armstütze, die einen mechanisch arbeitenden Steuermechanismus aufweist, ist jedoch, wie oben beschrieben, hinsichtlich der Funktionen Anheben, Absenken und Feststellen der Armauflage nachteilig.

Aus dem Dokument US 5,791,263 ist eine höhenverstellbare Auflage beispielsweise für eine Computertastatur offenbart. Die Auflage wird an einem Träger, beispielsweise einem Tisch, montiert und ist bezüglich dieses Trägers höhenverstellbar. Die Auflage ist mit einem Schwenkarm verbunden, der an seinem der Auflage abgewandten Ende an einer Schwenkachse befestigt ist. Ebenfalls mit der Schwenkachse befestigt ist ein Hebel, der beim Verschwenken des Schwenkarms um die Schwenkachse mit verschwenkt wird. An dem Hebel sind zwei Reibstangen angelenkt, die beim Verschwenken des Hebels um die Schwenkachse in eine Bewegung in Richtung ihrer Längsrichtung versetzt werden. Mit den Reibstangen wirkt ein Klemmteil zusammen, derart, dass bei einer auf die Auflage nach unten wirkenden Kraft das Klemmteil mit den Reibstangen klemmend in Eingriff kommt, so dass die Abwärtsbewegung der Auflage blockiert ist, während das Klemmteil bei einer Aufwärtsbewegung der Auflage mit den Reibstangen außer Eingriff kommt, wodurch die Aufwärtsbewegung der Auflage möglich ist. Um die Auflage auch abwärts bewegen zu können, ist mit dem Klemmteil ein Betätigungsmechanismus verbunden, der bei Betätigung das Klemmteil für die Abwärtsbewegung der Auflage mit den Reibstangen außer Eingriff bringt.

US 2005/051688 A1 offenbart einen lageverstellbaren Halter für einen Flachbildschirm. Der Halter weist eine Freilaufkupplung mit Bremswirkung auf. Ein mit der Halterplatte des Halters verbundener Schwenkarm ist mit einem drehbaren Kupplungsteil der Freilaufkupplung drehfest verbunden.

DE 30 32 415 A1 offenbart einen Gelenkarm zum Halten medizinischer Geräte, mit einer Freilaufkupplung ohne Bremswirkung, wobei verschwenkbare Arme der Tragstruktur drehfest mit den drehbaren Teilen der Freilaufkupplung gekoppelt sind.

Der Erfindung liegt daher die Aufgabe zugrunde, eine chirurgische Armstütze der eingangs genannten Art dahingehend weiterzubilden, dass sie einen hinsichtlich der vorstehend genannten Funktionen verbesserten Steuermechanismus aufweist.

Erfindungsgemäß wird diese Aufgabe hinsichtlich der eingangs genannten chirurgischen Armstütze dadurch gelöst, dass die Stange mit einer Freilaufkupplung zusammenwirkt, die ein erstes Kupplungselement und ein zweites Kupplungselement aufweist, wobei das erste Kupplungselement von der Stange angetrieben wird und bei einer Aufwärtsbewegung des zumindest einen Schwenkarms gegenüber dem zweiten Kupplungselement frei beweglich und bei einer Abwärtsbewegung des zumindest einen Schwenkarms das zweite Kupplungselement mit antreibt, und dass das zweite Kupplungselement mittels zumindest eines Bremselements gebremst ist.

Der Steuermechanismus der erfindungsgemäßen chirurgischen Armstütze ist demnach mit einer Freilaufkupplung versehen, die mit der Stange derart zusammenwirkt, dass sie eine unterschiedliche Gängigkeit des zumindest einen Schwenkarms in Abwärtsrichtung und in Aufwärtsrichtung gewährleistet. Dazu weist die Freilaufkupplung ein erstes Kupplungselement auf, das bei jeder Abwärts- und Aufwärtsbewegung des zumindest einen Schwenkarms von der Stange angetrieben wird. Bei einer Aufwärtsbewegung des zumindest einen Schwenkarms ist das erste Kupplungselement gegenüber dem zweiten Kupplungselement frei beweglich, so dass der Schwenkarm und damit die Armauflage leichtgängig bewegbar ist. Bei einer Abwärtsbewegung des zumindest einen Schwenkarms zum Absenken der Armauflage treibt dagegen das erste Kupplungselement das zweite Kupplungselement an, und weil dieses jedoch mittels eines Bremselements gebremst ist, ist die Abwärtsbewegung des zumindest einen Schwenkarms gegenüber der Aufwärtsbewegung schwergängiger. Dadurch wird verhindert, dass die Armauflage unkontrolliert absackt, da die Abwärtsbewegung durch das Bremselement gedämpft ist.

Eine solche Freilaufkupplung gemäß der Erfindung kann vorteilhafterweise aus Teilen aufgebaut werden, die einer Autoklavierung standhalten, ermöglicht aber gegenüber der bekannten Armstütze mit mechanischem Steuermechanismus eine verbesserte Betriebssicherheit und Handhabbarkeit.

In einer bevorzugten Ausgestaltung stehen das erste Kupplungselement und das zweite Kupplungselement bei der Abwärtsbewegung des zumindest einen Schwenkarms über einen Formschluss miteinander in Verbindung, während bei einer Aufwärtsbewegung des zumindest einen Schwenkarms der Formschluss durch eine Beabstandung des zweiten Kupplungselements von dem ersten Kupplungselement aufgehoben ist.

Der Vorteil dieser Maßnahme besteht darin, dass die unterschiedliche Gängigkeit der Verschwenkung des zumindest einen Schwenkarms in Aufwärts- und Abwärtsrichtung durch eine bloße Beabstandung des zweiten Kupplungselement vom ersten Kupplungselement bewerkstelligt wird, durch die der Formschluss zwischen dem ersten und zweiten Kupplungselement aufgehoben wird. Der Formschluss zwischen dem ersten und zweiten Kupplungselement bei der Abwärtsbewegung des zumindest einen Schwenkarms stellt sicher, dass zwischen dem ersten und zweiten Kupplungselement bei dieser Bewegung kein Schlupf besteht und somit die Bremswirkung des zweiten Kupplungselements für eine kontrollierte Abwärtsbewegung des zumindest einen Schwenkarms gewährleistet ist.

In einer weiteren bevorzugten Ausgestaltung sind das erste Kupplungselement und das zweite Kupplungselement um eine gemeinsame Drehachse drehbar und weisen auf ihren einander zugewandten Stirnseiten eine komplementäre Schrägverzahnung auf, die in der einen Drehrichtung des ersten Kupplungselements ein Abstoßen des zweiten Kupplungselements und in der entgegengesetzten Richtung des ersten Kupplungselements eine Drehbewegung des zweiten Kupplungselements bewirkt.

In dieser Ausgestaltung sind das erste Kupplungselement und das zweite Kupplungselement demnach bezüglich der gemeinsamen Drehachse hintereinander angeordnet, was den Bauraum der Freilaufkupplung verringert. Die stirnseitige komplementäre Schrägverzahnung stellt nun eine konstruktiv vorteilhaft einfache Maßnahme dar, in der einen Drehrichtung des Kupplungselements den Formschluss mit dem zweiten Kupplungselement herzustellen, während bei einer umgekehrten Drehrichtung des ersten Kupplungselements dessen Schrägverzahnung das zweite Kupplungselement ohne das Erfordernis eines manuellen Eingriffes selbsttätig abstößt, um den Freilauf des ersten Kupplungselements gegenüber dem zweiten Kupplungselement zu gewährleisten.

Dabei ist es weiterhin bevorzugt, wenn das zweite Kupplungselement in Richtung der Drehachse axial beweglich, und in Richtung zum ersten Kupplungselement hin vorgespannt ist.

Diese Maßnahme hat den Vorteil, dass nach jeder Aufwärtsbewegung des zumindest einen Schwenkarms das zweite Kupplungselement mit dem ersten Kupplungselement automatisch wieder in formschlüssigen Eingriff kommt, wodurch nach Beenden jedes Anhebens der Armauflage die Bremse wieder aktiviert ist.

In einer weiteren bevorzugten Ausgestaltung verläuft die gemeinsame Drehachse zumindest annähernd senkrecht zur axialen Bewegungsrichtung der Stange.

Der Vorteil dieser Maßnahme besteht in einer platzsparenden Bauweise, da die Freilaufkupplung in dieser Ausgestaltung quer zu dem zumindest einen Schwenkarm angeordnet ist.

In einer weiteren bevorzugten Ausgestaltung treibt die Stange das erste Kupplungselement über eine Getriebeanordnung an.

Der Vorteil dieser Maßnahme besteht darin, dass eine Getriebeanordnung bei entsprechender Auslegung eine für den Antrieb der Freilaufkupplung über die Stange erforderliche und angepasste Kraftübertragung bereitstellen kann.

Dabei ist es weiter bevorzugt, wenn die Getriebeanordnung zumindest ein Zahnrad aufweist, das mit einer Verzahnung der Stange kämmt.

Die Ausgestaltung der Getriebeanordnung mit zumindest einem Zahnrad ist im Sinne einer Autoklavierbarkeit des Steuermechanismus und damit der gesamten Armstütze vorteilhaft.

Weiterhin ist es dabei bevorzugt, wenn die Getriebeanordnung zumindest zwei miteinander kämmende Zahnräder aufweist, die vorzugsweise einen verschiedenen Durchmesser aufweisen.

Die Verwendung zumindest zweier miteinander kämmender Zahnräder, von denen dann eines mit der Zahnstange kämmt und das andere mit dem ersten Kupplungselement verbunden ist, hat den Vorteil, dass durch die unterschiedlichen Durchmesser der beiden Zahnräder ein geeignetes Drehzahlübersetzungsverhältnis gewählt werden kann. Beispielsweise kann ein Zahnrad mit größerem Durchmesser mit der Verzahnung der Stange kämmen, und das zweite Zahnrad, das beispielsweise auf der Welle des ersten Kupplungselements sitzt, hat demgegenüber einen kleineren Durchmesser.

In einer weiteren bevorzugten Ausgestaltung ist die durch das Bremselement bereitgestellte Bremswirkung einstellbar.

Diese Maßnahme hat den Vorteil, dass die Bremswirkung an die jeweiligen Erfordernisse der Person, die die Armstütze benutzt, angepasst werden kann. Insbesondere kann im Fall, dass die Abwärtsbewegung der Armauflage durch eine Federkraft bewirkt oder unterstützt wird, die Bremswirkung an die Federkraft angepasst werden, um eine gewünschte Absenkgeschwindigkeit der Armauflage einzustellen.

In einer weiteren bevorzugten Ausgestaltung ist das Bremselement aus einem elastischen Material gefertigt, und eine Spanneinrichtung steht mit dem Bremselement in Wirkverbindung, um den Anpressdruck des Bremselements an das zweite Kupplungselement zu variieren.

Diese Maßnahme stellt eine konstruktiv vorteilhaft einfache Möglichkeit dar, die Bremswirkung des Bremselements auf das zweite Kupplungselement einzustellen. Die Spanneinrichtung kann beispielsweise und bevorzugt eine Spannzange und ein Einstellorgan für die Spannzange aufweisen. Als elastisches Material für das Bremselement wird vorzugsweise ein autoklavierbares Material verwendet, beispielsweise Silikon.

In einer weiteren bevorzugten Ausgestaltung ist der Stange zumindest eine Feder zugeordnet, die bei einer Aufwärtsbewegung des zumindest einen Schwenkarms gespannt und bei einer Abwärtsbewegung entspannt wird.

Der Vorteil dieser Maßnahme besteht darin, dass die Stange in Abwärtsrichtung der Schwenkbewegung des zumindest einen Schwenkarms vorgespannt ist. Bei der aufwärts gerichteten Schwenkbewegung des zumindest einen Schwenkarms und damit zum Anheben der Armauflage wird daher gegen die Kraft der Feder gearbeitet. Die Vorspannung der Stange sollte jedoch nicht so stark sein, dass die Aufwärtsbewegung schwergängig wird. Die Vorspannung der Stange mittels einer Feder in Abwärtsrichtung hat den Vorteil, dass nach Lösen einer Sperre für die Stange sich die Armauflage selbsttätig, durch die Bremswirkung der Freilaufkupplung jedoch kontrolliert, absenkt.

Dazu ist es weiterhin bevorzugt, wenn die zumindest eine Feder so ausgelegt ist, dass der zumindest eine Schwenkarm durch die Kraft der Feder gegen die von dem Bremselement erzeugte Bremswirkung in Abwärtsrichtung verschwenkt wird.

Die Vorspannung der Feder ist vorzugsweise einstellbar.

Des Weiteren ist in einer bevorzugten Ausgestaltung vorgesehen, dass die Tragstruktur zumindest einen zweiten Schwenkarm aufweist, der zumindest näherungsweise parallel zu dem ersten Schwenkarm verläuft und einerseits an der Basis um eine von der Schwenkachse des ersten Schwenkarmes vertikal beabstandete Schwenkachse angelenkt und andererseits mit der Armauflage verbunden ist, und dass die Stange teilweise in einem Zylinder relativ zu diesem beweglich aufgenommen ist und der Zylinder an dem zweiten Schwenkarm befestigt ist.

Die Tragstruktur der erfindungsgemäßen chirurgischen Armstütze ist bei dieser Ausgestaltung demnach mit zwei parallelogrammartig angeordneten Schwenkarmen ausgebildet, ähnlich zu der eingangs genannten bekannten Armstütze. Eine solche parallelogrammartige Struktur der Schwenkarme erhöht die Gesamtstabilität der Armstütze, insbesondere wenn der Steuermechanismus, der die Stange mit Zylinder umfasst, unmittelbar zwischen die beiden Schwenkarme geschaltet ist. Im Rahmen der Erfindung ist es jedoch ebenso denkbar, nur einen Schwenkarm zu verwenden, und die Stange über den Zylinder an der Basis zu befestigen.

In einer weiteren bevorzugten Ausgestaltung ist für die Stange eine lösbare Raste vorgesehen, die die Stange axial unbeweglich sperrt und nach Lösen freigibt.

Diese Maßnahme erhöht vorteilhafterweise die Betriebssicherheit der chirurgische Armstütze, da die Raste gewährleistet, dass ein unerwünschtes Absinken der Armauflage vermieden wird, indem die Raste die axiale Beweglichkeit der Stange und damit die Verschwenkbarkeit des zumindest eines Schwenkarmes sperrt. Damit kann sich die die Armstütze bedienende Person sicher auf der Armauflage abstützen, ohne dass die Gefahr besteht, dass die Armauflage absinkt. Ein Absenken der Armauflage ist bei dieser Ausgestaltung erst nach Lösen der Raste möglich.

Dabei ist es weiter bevorzugt, wenn die Raste durch einen Riegel gebildet wird, der eine Durchführung für die Stange aufweist, wobei der Riegel um eine Kippachse in eine erste Kippstellung, in der die Stange schräg durch die Durchführung hindurchgeht und dadurch an der Durchführung geklemmt ist, und in eine zweite Kippstellung bewegbar ist, in der die Stange im Wesentlichen senkrecht durch die Durchführung hindurchgeht und dadurch axial beweglich ist.

Der Vorteil einer Ausgestaltung der lösbaren Raste in der angegebenen Weise hat den Vorteil, dass die Sperrung bzw. Versiegelung der Stange stufenlos bezüglich der eingestellten Höhe der Armauflage ist. Somit kann auch die Armauflage zwischen einer niedrigsten Stellung und einer höchsten Stellung in jeder Zwischenhöhe arretiert werden.

Dabei ist es weiter bevorzugt, wenn die Armauflage relativ zu dem zumindest einen Tragarm beweglich ist, und wenn die Raste mit der Armauflage derart verbunden ist, dass durch geringfügiges Anheben der Armauflage relativ zum zumindest einen Schwenkarm die Raste aus ihrer Sperrstellung in die Entsperrstellung und durch geringfügiges Absenken der Armauflage relativ zu dem zumindest einen Schwenkarm in die Sperrstellung überführt wird.

Der Vorteil dieser Maßnahme besteht darin, dass die Raste durch einfache Handhabung gelöst werden kann, indem nämlich die Armauflage geringfügig angehoben wird. Die Aktivierung der Raste erfolgt einfach durch geringfügiges Absenken der Armauflage relativ zu dem zumindest einen Schwenkarm, was bereits durch die Gewichtskraft der Armauflage im Wesentlichen selbsttätig erfolgt. Die Raste kann zusätzlich vorzugsweise in die Sperrstellung vorgespannt sein.

In einer weiteren bevorzugten Ausgestaltung ist die Armauflage so ausgebildet, dass sie ein Anheben der Armauflage mit demselben Arm ermöglicht, der sich auf der Armauflage abstützt.

Der Vorteil dieser Maßnahme besteht darin, dass auch das Lösen der Raste vorteilhaft einfach mit dem Arm durchgeführt werden kann, der sich gerade in der Armauflage abstützt. Auf diese Weise ist eine vollständige Bedienung aller Freiheitsgrade der Armauflage mit demjenigen Arm möglich, der sich gerade auf der Armauflage abstützt, ohne dass der Benutzer die Hand dieses oder des anderen Armes zur Betätigung der chirurgischen Armstütze benötigt. Es ist somit möglich, alle Bewegungen der Armauflage mit dem in der Armauflage aufgenommenen Arm zu steuern.

In einer konstruktiv einfachen Ausgestaltung der Armauflage ist diese als im Wesentlichen C-förmige Schale ausgebildet, so dass der in der Armauflage aufgenommene Arm auch auf seiner Oberseite zumindest teilweise von der Armauflage umspannt ist. Die C-förmige Ausgestaltung hat darüber hinaus den Vorteil, dass der Arm von der Seite in die Armauflage eingeführt werden kann.

Weitere Vorteile und Merkmale ergeben sich aus der nachfolgenden Beschreibung und der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese hiernach näher beschrieben. Es zeigen:
- Figur 1: eine perspektivische Gesamtdarstellung einer chirurgischen Armstütze;
- Figur 2: eine Seitenansicht der Armstütze in Figur 1 in gegenüber Figur 1 verkleinertem Maßstab und mit einer vergrößert dargestellten Einzelheit der Armstütze;
- Figuren 3a) und b): jeweils zwei Kupplungselemente einer Freilaufkupplung der Armstütze in Figuren 1 und 2, wobei Figur 3a) die beiden Kupplungselemente in einer ersten Betriebsstellung und Figur 3b) in einer zweiten Betriebsstellung zeigt;
- Figuren 4a) und b): Längsschnitte der Freilaufkupplung der Armstütze in Figuren 1 und 2, wobei Figur 4a) die Freilaufkupplung in einem Figur 3a) entsprechenden Betriebszustand und Figur 4b) in einem Figur 3b) entsprechenden Betriebszustand dargestellt ist;
- Figuren 5a) und b): weitere Einzelheiten der chirurgischen Armstütze in Figuren 1 und 2 in zwei unterschiedlichen Betriebszuständen;
- Figuren 6a) und b): die chirurgische Armstütze in Figuren 1 und 2 unter Weglassung von Teilen gegenüber Figuren 1 und 2, wobei Figur 6a) die Armstütze in einem ersten Betriebszustand und Figur 6b) in einem zweiten Betriebszustand zeigt;
- Figuren 7a) und b): die chirurgische Armstütze unter Weglassung von Teilen gegenüber Figuren 1 und 2 in jeweils einer Seitenansicht in unterschiedlichen Betriebszuständen der Armstütze; und
- Figuren 8a) und b): weitere Einzelheiten der chirurgischen Armstütze in Figuren 1 und 2 in zwei unterschiedlichen Betriebszuständen.

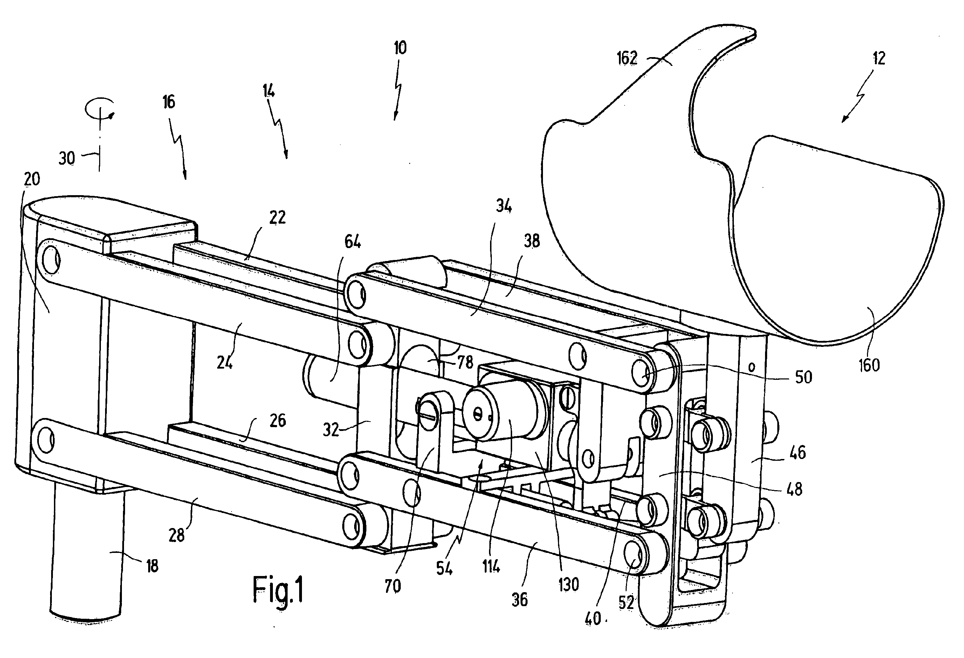

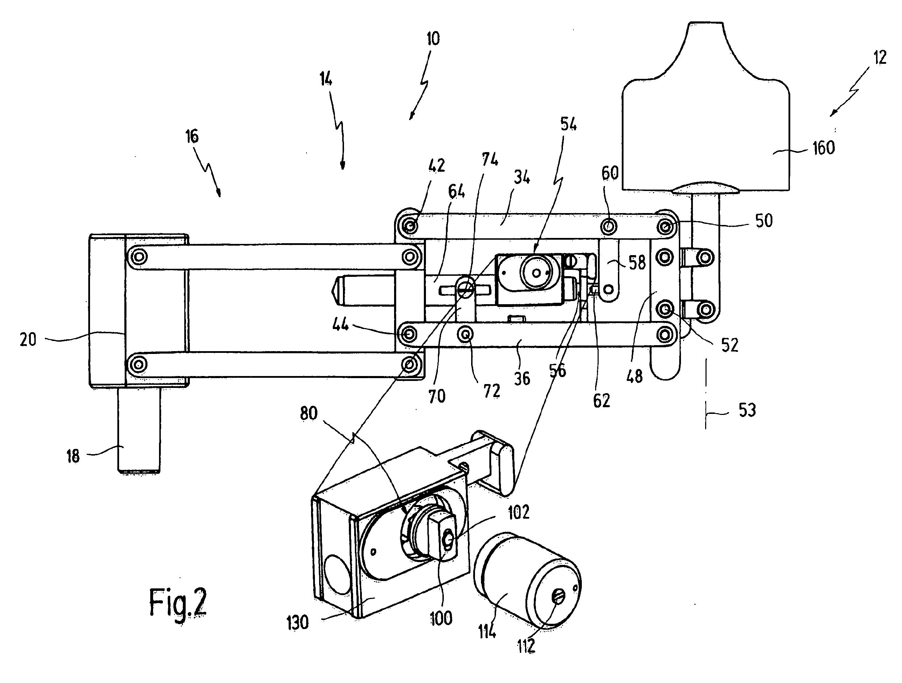

In Figuren 1 und 2 ist eine mit dem allgemeinen Bezugszeichen 10 versehene chirurgische Armstütze dargestellt. Die Armstütze 10 dient dazu, während einer Operation den Arm eines Chirurgen oder einer chirurgischen Assistenz in einer benutzergewählten Position zu stützen, und diese Position auch zu verändern.

Die Armstütze 10 weist allgemein eine Armauflage 12 und eine Tragstruktur 14 für die Armauflage 12 auf.

Die Tragstruktur 14 weist eine Basis 16 auf, die in dem gezeigten Ausführungsbeispiel einen Stiel 18 aufweist, der zur Montage der Armstütze 10 an einem nicht dargestellten Stativ oder einem nicht dargestellten Operationstisch dient. Oberhalb des Stiels 18 ist dieser mit einem Tragblock 20 verbunden, der wiederum zumindest einen, in dem gezeigten Ausführungsbeispiel vier Tragarme 22, 24, 26 und 28 trägt. Die Tragarme 22 bis 28 sind in dem gezeigten Ausführungsbeispiel relativ zu dem Tragblock 20 unbeweglich, könnten jedoch ebenso um im Wesentlichen horizontale Schwenkachsen feststellbar verschwenkbar an dem Tragblock 20 montiert sein, um eine grobe Voreinstellung der Höhe der Armauflage 12 zu ermöglichen.

Die Basis 16 kann insbesondere um eine im Wesentlichen vertikale Achse 30 drehbar sein, so dass die Armauflage 12 in einer im Wesentlichen horizontalen Ebene entsprechend der eingestellten Höhe verschwenkt werden kann.

Die Basis weist an den Enden der Tragarme 22 bis 28, die von dem Tragblock 20 abgewandt sind, eine Strebe 32 auf, die sich im Wesentlichen vertikal erstreckt und an der die vier Tragarme 22 bis 28 befestigt sind.

Die Tragstruktur 14 weist weiterhin zumindest einen, in dem gezeigten Ausführungsbeispiel vier Schwenkarme 34, 36, 38, 40 auf, die zusammen eine parallelogrammartige Struktur bilden.

Mit ihren den Tragarmen 22 bis 28 zugewandten Enden sind die Schwenkarme 34 bis 40 um jeweils eine zumindest näherungsweise horizontale Schwenkachse verschwenkbar angelenkt, und zwar an der Strebe 32 der Basis 16. Die Schwenkarme 34 und 38 verlaufen dabei parallel zueinander und sind um eine gemeinsame Schwenkachse 42 verschwenkbar an der Basis 16 angelenkt, und die Schwenkarme 36 und 40, die ebenfalls parallel zueinander verlaufen, sind um eine von der Schwenkachse 42 vertikal beabstandete Schwenkachse 44 verschwenkbar.

An ihrem den Schwenkachsen 42 bzw. 44 gegenüberliegenden Ende sind die Schwenkarme 34 und 38 bzw. 36 und 40 mit der Armauflage 12 verbunden. In dem gezeigten Ausführungsbeispiel ist die Armauflage 12 mittels eines im Wesentlichen vertikal angeordneten Stützelements 46 an einer ebenfalls im Wesentlichen vertikal angeordneten Strebe 48 befestigt, mit der auch die den Schwenkachsen 42 bzw. 44 gegenüberliegenden Enden der Schwenkarme 34 bis 40 an Achsen 50 und 52 gelenkig verbunden sind.

Die Armauflage 12 kann um eine Achse 53, vorzugsweise um 360°, gedreht werden.

Über eine Verschwenkung der Schwenkarme 34 bis 40 um die Schwenkachsen 42 bzw. 44 in Aufwärts- oder Abwärtsrichtung wird die Armauflage 12 nach oben bzw. nach unten verstellt. Um ein Anheben der Armauflage 12 nach oben, ein kontrolliertes Absenken der Armauflage 12 nach unten sowie ein Feststellen der Armauflage 12 in einer eingestellten Höhenposition zu gewährleisten, ist ein Steuermechanismus 54 vorgesehen, der nachfolgend beschrieben wird.

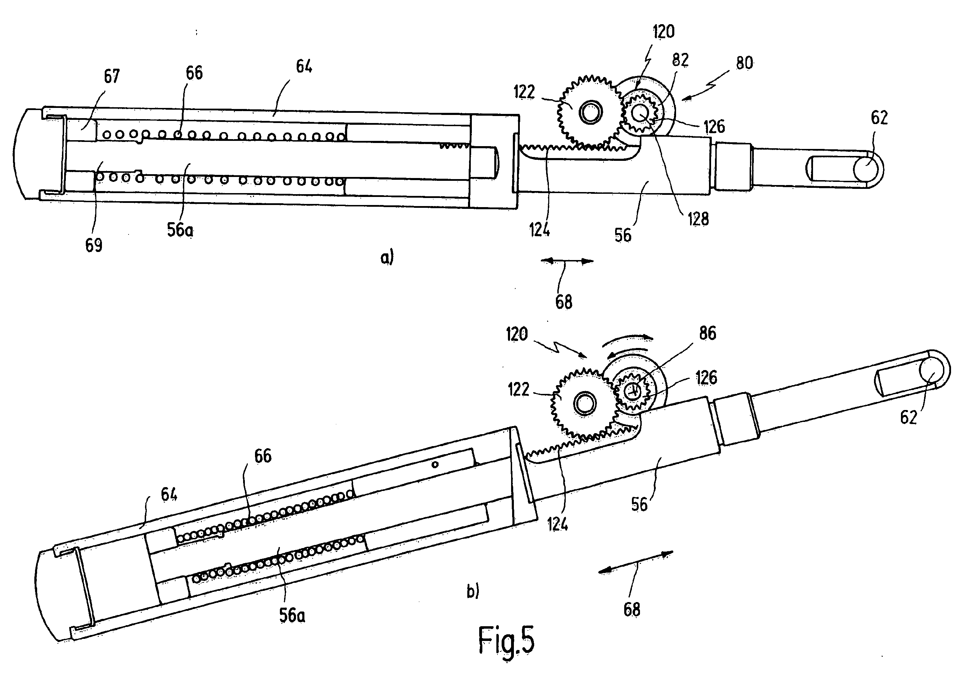

Zunächst weist der Steuermechanismus 54 mit Bezug auf die Figuren 2 und 5a), b), 6b) sowie 8a), b) eine Stange 56 auf, die mit den Schwenkarmen 34 und 38 verbunden ist. Um eine günstige Winkellage der Stange 56 relativ zu den Schwenkarmen 34, 38 zu erreichen, ist mit den Schwenkarmen 34 und 38 ein Beabstandungselement 58 verbunden, und zwar an einer Befestigungsstelle 60, und an dem der Befestigungsstelle 60 gegenüberliegenden Ende des Verbindungselements 58 ist die Stange 56 mit diesem, über ein Auge 62, gelenkig verbunden.

Die Stange 56 ist gemäß Figuren 5a) und b) teilweise in einem Zylinder 64 aufgenommen, und zwar mit einem Abschnitt 56a der Stange 56, wobei sich die Stange 56 relativ zu dem Zylinder 64 in Längsrichtung der Stange 56 axial bewegen kann.

In dem Zylinder 64 ist ferner zumindest eine Feder 66 angeordnet. Die Vorspannung der Feder 66 kann mittels eines Widerlagers 67, das mit einem Ende 69 der Stange 56 vorzugsweise über einen Gewindegang verbunden ist, verstellt bzw. eingestellt werden.

Bei einer Verschwenkung der Schwenkarme 34 bis 40 um die Schwenkachsen 42 bzw. 44 führt die Stange 56 (neben einer Kippung) eine in ihrer Längsrichtung gerichtete axiale Bewegung relativ zu dem Zylinder 64 aus. Diese Längsbewegung ist in Figuren 5a) und b) mit einem Doppelpfeil 68 veranschaulicht, wobei bei einer aufwärts gerichteten Schwenkbewegung der Schwenkarme 34 bis 40 die Stange 56 nach rechts in Figuren 5a) und b) bewegt wird, während bei einer abwärts gerichteten Schwenkbewegung der Schwenkarme 34 bis 40 die Stange 56 nach links in Figuren 5a) und b) bewegt wird. Figur 5a) zeigt die Stange 56 in ihrer maximal in den Zylinder 64 eingeschobenen Position. Dies entspricht der Schwenkstellung der Schwenkarme 34 bis 40 in Figuren 1 und 2, in der die Armauflage 12 maximal abgesenkt ist. In dieser Stellung ist die Feder 66 entspannt, und bei einer aufwärts gerichteten Schwenkbewegung der Schwenkarme 34 bis 40, bei der die Stange 56 in Richtung aus dem Zylinder heraus bewegt wird, wird die Feder 66 gespannt, wie in Figur 5b) dargestellt ist. Figur 5b) zeigt die maximal ausgefahrene Stellung der Stange 56.

Der Zylinder 64 ist mit den Schwenkarmen 36 und 40 verbunden, und zwar über ein bügelförmiges Element 70 (vgl. Figur 1), das mit den Schwenkarmen 36 und 40 an Befestigungsstellen 72 und mit dem Zylinder 64 über ein Gelenk 74 verbunden ist. Über das Gelenk 74 wird eine relative Kippbewegung zwischen dem Zylinder 64 und dem bügelförmigen Element 70 ermöglicht, wobei jedoch der Zylinder 64 in seiner Längsrichtung unbeweglich an dem bügelförmigen Element gehalten ist.

Gemäß Figur 1 ist in der Strebe 32 der Basis 16 eine Durchbrechung 78 vorhanden, durch die der Zylinder 64 hindurchragt, wobei die Durchbrechung 78 ausreichend bemessen ist, so dass der Zylinder 64 die beim Verschwenken der Schwenkarme 34 bis 40 erforderlichen Kippbewegungen ausführen kann.

Der Steuermechanismus 54 weist weiterhin eine Freilaufkupplung 80 auf, mit der die Stange 56 zusammenwirkt, um eine unterschiedliche Gängigkeit der Schwenkbewegungen der Schwenkarme 34 bis 40 in Aufwärts- und Abwärtsrichtung und damit der Höhenverstellung der Armauflage 12 zu bewirken.

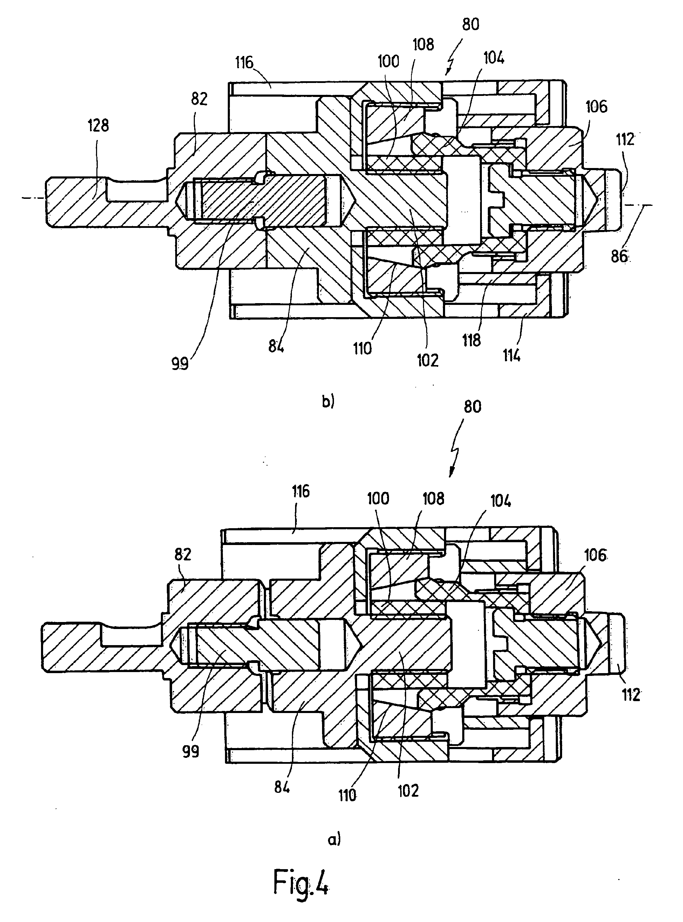

In Figuren 4a) und b) ist die Freilaufkupplung 80 in Alleinstellung gezeigt.

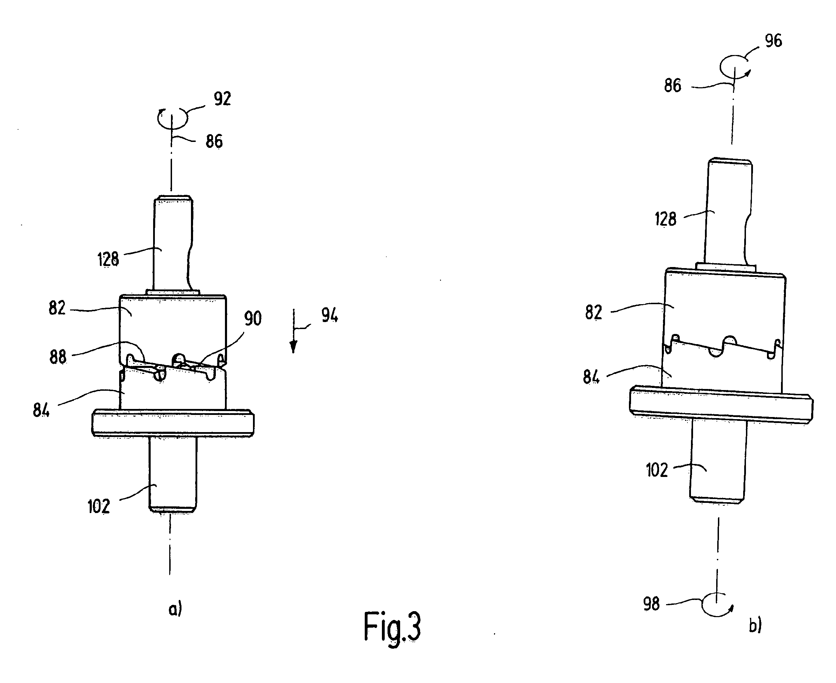

Die Freilaufkupplung 80 weist ein erstes Kupplungselement 82 und ein zweites Kupplungselement 84 auf, die in Figuren 3a) und b) in Alleinstellung gezeigt sind.

Die Kupplungselemente 82 und 84 sind in Form von Rädern ausgebildet, die um eine gemeinsame Drehachse 86 drehbar und bezüglich dieser Drehachse 86 konzentrisch und hintereinander angeordnet sind.

Das erste Kupplungselement 82 und das zweite Kupplungselement 84 weisen bezüglich der Drehachse 86 stirnseitig eine komplementäre Schrägverzahnung 88 (Kupplungselement 82) und 90 (Kupplungselement 84) auf. Die Form der Schrägverzahnungen 88 und 90 ist in Figur 3 dargestellt.

Bei einer Drehung des ersten Kupplungselements 82 um die Drehachse 86 in Richtung eines Pfeiles 92 bewirkt die Schrägverzahnung, dass die Stirnseite des Kupplungselements 82 auf dem Kupplungselement 84 gleitet, so dass das zweite Kupplungselement 84 bei einer solchen Drehung des ersten Kupplungselements 82 nicht in Drehung versetzt wird. Aufgrund der Form der Schrägverzahnung 88 und 90 wird bei einer Drehung des Kupplungselements 82 in Richtung des Pfeils 92 außerdem das zweite Kupplungselement 84 in Richtung eines Pfeiles 94 in Figur 3a) abgestoßen, so dass das zweite Kupplungselement 84 durch die Drehbewegung des ersten Kupplungselements 82 in Richtung des Pfeiles 92 von dem ersten Kupplungselement 82 beabstandet wird, und zwar in dem Maß, wie in Figur 3a) dargestellt ist. Das zweite Kupplungselement 84 ist zu diesem Zweck in Richtung der Drehachse 86 axial beweglich.

Gemäß Figur 4 sind das erste Kupplungselement 82 und das zweite Kupplungselement 84 über einen Wellenstift 99 relativ zueinander zentriert, wobei der Wellenstift 99 mit dem ersten Kupplungselement 82 fest verbunden ist, während das zweite Kupplungselement 84 relativ zu dem Wellenstift 99 um die Drehachse 86 drehbar und außerdem in Richtung der Drehachse 86 axial verschiebbar ist.

Bei einer Drehung des ersten Kupplungselements 82 um die Drehachse 86 in Richtung eines Pfeiles 96, das heißt in entgegengesetzter Drehrichtung wie in Figur 3a), kommt das erste Kupplungselement 82 über die Schrägverzahnung 88 mit der Schrägverzahnung 90 des zweiten Kupplungselements 84 formschlüssig in Eingriff, wodurch das zweite Kupplungselement 84 von dem ersten Kupplungselement 82 in Drehung versetzt wird, wie in Figur 3b) mit einem Pfeil 98 veranschaulicht ist.

Figur 3a) stellt demnach die Freilaufstellung der Freilaufkupplung 80 dar, während Figur 3b) die Betriebsstellung der Kupplung 80 zeigt, in der das zweite Kupplungselement 84 mitläuft.

Die Betriebsstellung in Figur 4a) entspricht der Betriebsstellung in Figur 3a), und die Betriebsstellung in Figur 4b) entspricht der Betriebsstellung in Figur 3b).

Weiter mit Bezug auf Figur 4 wirkt das zweite Kupplungselement 84 mit einem Bremselement 100 zusammen, das eine Drehung des zweiten Kupplungselements 84 um die Drehachse 86 durch Reibschluss zwischen dem Bremselement 100 und dem zweiten Kupplungselement 84 bremst. Das Bremselement 100, das in Figur 2 in dem vergrößerten Ausschnitt perspektivisch dargestellt ist, ist aus einem elastischen Material gefertigt, beispielsweise aus Silikon.

Das Bremselement 100 ist auf einem Wellenfortsatz 102 des zweiten Kupplungselements 84 angeordnet, wobei der Wellenfortsatz 102 relativ zu dem nicht drehbaren Bremselement 100 drehbar ist.

Durch einen einstellbaren Anpressdruck des Bremselements 100 an dem Wellenfortsatz 102 des zweiten Kupplungselements 84 lässt sich die Bremswirkung des Bremselements 100 einstellen. Dazu ist eine Spanneinrichtung vorgesehen, die eine Spannzange 104 und einen axialen Verstellmechanismus in Form einer Einstellhülse 106 aufweist, wobei die Spannzange mit einem Gegenhalter 108 zusammenwirkt, der eine sich verjüngende Rampe 110 aufweist, so dass bei einem durch die Einstellhülse 106 vermittelten Vorschieben der Spannzange 104 diese das Bremselement 100 mit zunehmendem Druck an den Wellenfortsatz 102 andrückt. Die Einstellhülse 106 ist gemäß dem vergrößerten Ausschnitt in Figur 2 mit einem geschlitzten Kopf 112 versehen, um die Einstellhülse 106 zum Einstellen der Bremswirkung des Bremselements 100 mit einem Schraubendreher betätigen zu können.

Die Freilaufkupplung 80 weist ferner ein Gehäuseteil 114 auf, das über einen Führungsschlitz oder eine Führungsnut 116 außerdem die axiale Führung des zweiten Kupplungselements 84 zusammen mit dem Bremselement 100 und der zuvor beschriebenen Spanneinrichtung ermöglicht. Die axiale Beweglichkeit der zuvor genannten Teile ist, wie bereits beschrieben, erforderlich, damit das erste Kupplungselement 82 in der Drehrichtung gemäß dem Pfeil 92 in Figur 3a) das zweite Kupplungselement 84 und die damit verbundenen Teile abstoßen kann, wie in Figur 4a) dargestellt ist.

Damit das zweite Kupplungselement 84 bei einer Abwärtsbewegung der Schwenkarme 34 bis 40 mit seiner Schrägverzahnung 90 wieder selbsttätig mit der Schrägverzahnung 88 des ersten Kupplungselements 82 formschlüssig in Eingriff kommen kann, ist das zweite Kupplungselement 84 über eine Feder 118 in Richtung zu dem ersten Kupplungselement 82 hin vorgespannt. Wie bereits erwähnt, bewegen sich auch das Bremselement 100 und die Spanneinrichtung für das Bremselement 100 mit dem zweiten Kupplungselement 84 axial mit.

Nachfolgend wird nun die Wirkverbindung der Stange 56 mit der Freilaufkupplung 80 beschrieben.

Die Stange 56 treibt bei ihrer Längsbewegung in Richtungen gemäß dem Doppelpfeil 68 in Figuren 5a) und b) die Freilaufkupplung 80 an, genauer gesagt treibt die Stange 56 stets das erste Kupplungselement 82 an. Zum Antrieb des ersten Kupplungselements 82 ist die Stange 56 mit dem ersten Kupplungselement 82 über eine Getriebeanordnung 120 verbunden, wie sie in Figuren 5a) und b) dargestellt ist.

Die Getriebeanordnung 120 weist ein erstes Zahnrad 122 auf, das mit einer Verzahnung 124 an einem Teilabschnitt der Stange 56 kämmend in Eingriff steht. Das Zahnrad 122 steht seinerseits mit einem zweiten Zahnrad 126 kämmend in Eingriff, das auf einem Wellenfortsatz 128 des ersten Kupplungselements 82 angeordnet und mit dem ersten Kupplungselement 82 drehfest verbunden ist. Das erste Zahnrad 122 weist einen größeren Durchmesser auf als das zweite Zahnrad 126, so dass sich ein Drehzahlübertragungsverhältnis von größer als 1 ergibt.

Wie aus Figuren 5a) und b) hervorgeht, ist die Freilaufkupplung 80 bezüglich der Stange 56 so orientiert, dass die Drehachse 86 der Kupplungselemente 82 und 84 gemäß dem Doppelpfeil 68 senkrecht zur axialen Bewegungsrichtung der Stange 56 gerichtet ist.

Die Getriebeanordnung 120 zusammen mit der Freilaufkupplung 80 ist in einem Hauptgehäuse 130 aufgenommen, das in Figur 2 in dem vergrößerten Ausschnitt isoliert dargestellt ist, und das an dem Zylinder 64 der Stange 56 befestigt ist. Das Hauptgehäuse 130 umfast auch das Gehäuseteil 114, das in Figur 2 in dem vergrößerten Ausschnitt abgenommen dargestellt ist.

Mit Bezug auf Figur 5b) wird der Antrieb der Freilaufkupplung 80 durch die Stange 56 beschrieben. Wird die Stange 56 in Richtung aus dem Zylinder 64 heraus bewegt, wie dies bei einer Aufwärtsverschwenkung der Schwenkarme 34 bis 40 der Fall ist, wird das erste Zahnrad 122 in der Darstellung entgegen dem Uhrzeigersinn in Drehung versetzt, wodurch wiederum das zweite Zahnrad 126 in eine Drehung im Uhrzeigersinn versetzt wird. Damit dreht sich das erste Kupplungselement 82 in Richtung des Pfeiles 92 in Figur 3a), so dass das erste Kupplungselement 82 frei läuft, das heißt das zweite Kupplungselement 84 nicht mit angetrieben wird.

Bei einer entgegengesetzten Bewegung der Stange 56 zum Abwärtsverschwenken der Schwenkarme 34 bis 40 ist die Drehrichtung der Zahnräder 122 bis 126 gerade umgekehrt, so dass das erste Kupplungselement 82 sich in Richtung des Pfeiles 96 gemäß Figur 3b) dreht. In dieser Drehrichtung besteht Formschluss zwischen der Schrägverzahnung 88 und der Schrägverzahnung 90, wodurch das zweite Kupplungselement 84 ebenfalls in Drehung versetzt wird. Da aber die Drehung des zweiten Kupplungselements 84 durch das Bremselement 100 reibungsbehaftet ist, ist diese Bewegung der Stange 56 und damit die abwärts gerichtete Schwenkbewegung der Schwenkarme 34 bis 40 gebremst bzw. gedämpft.

Auf diese Weise lässt sich die Armauflage 12 der Armstütze 10 zwischen zwei Endstellungen nach oben und nach unten bewegen, wobei die Aufwärtsbewegung der Armauflage 12 leichtgängig ist und die Abwärtsbewegung der Armauflage 12 aufgrund der Bremswirkung und je nach Einstellung der Bremswirkung schwergängiger ist und damit kontrolliert erfolgt. Der Aufwärtsbewegung der Armauflage 12 ist lediglich die Kraft der Feder 66 entgegengerichtet, die sich bei der Aufwärtsbewegung der Armauflage 12 mehr und mehr spannt (vgl. Figur 5b)).

Die Feder 66 ist vorzugsweise so ausgelegt, dass sie ein kontrolliertes Absenken der Armauflage 12 gegen die Bremskraft des Bremselements ohne zusätzliche Kraftausübung des Benutzers ermöglicht.

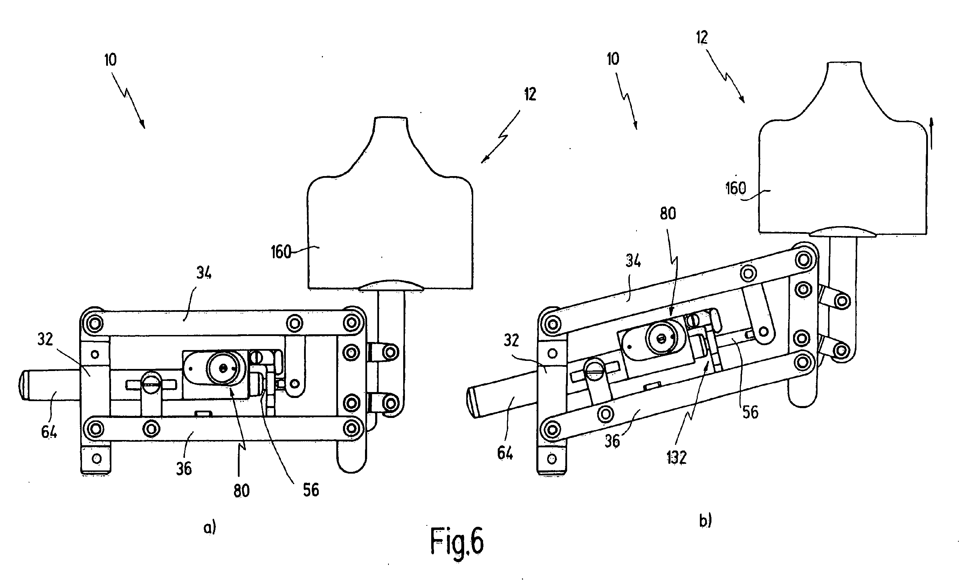

In Figur 6a) ist ein Betriebszustand der Armstütze 10 dargestellt, der die niedrigste Stellung der Armauflage 12 repräsentiert, in der die Schwenkarme 34 bis 40 nach unten verschwenkt und die Stange 56 in den Zylinder 64 eingefahren ist.

Figur 6b) zeigt demgegenüber einen Betriebszustand der Armstütze 10, in dem die Armauflage 12 gegenüber der Stellung in Figur 6a) angehoben ist. Die Stange 56 ist entsprechend ein Stück aus dem Zylinder 64 herausgezogen.

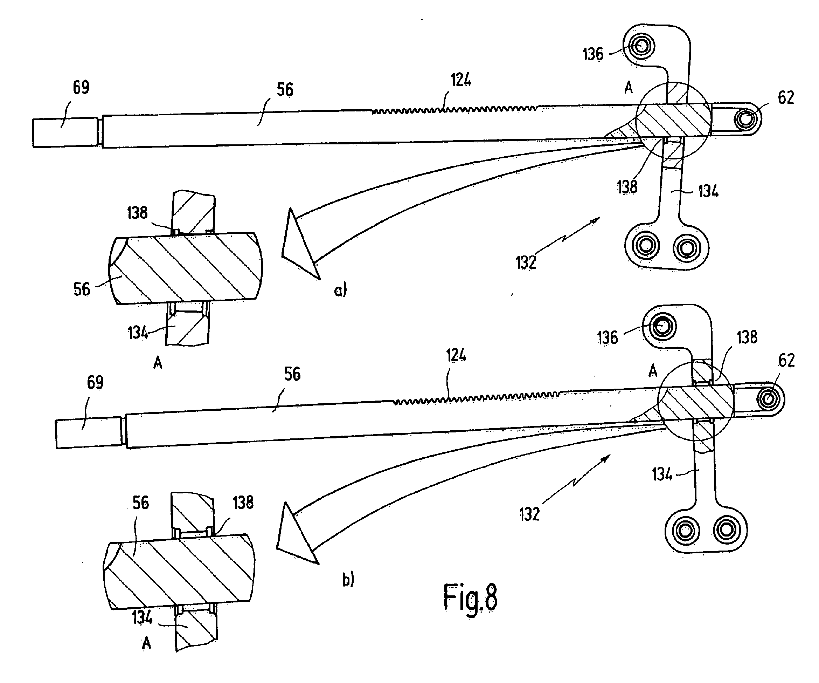

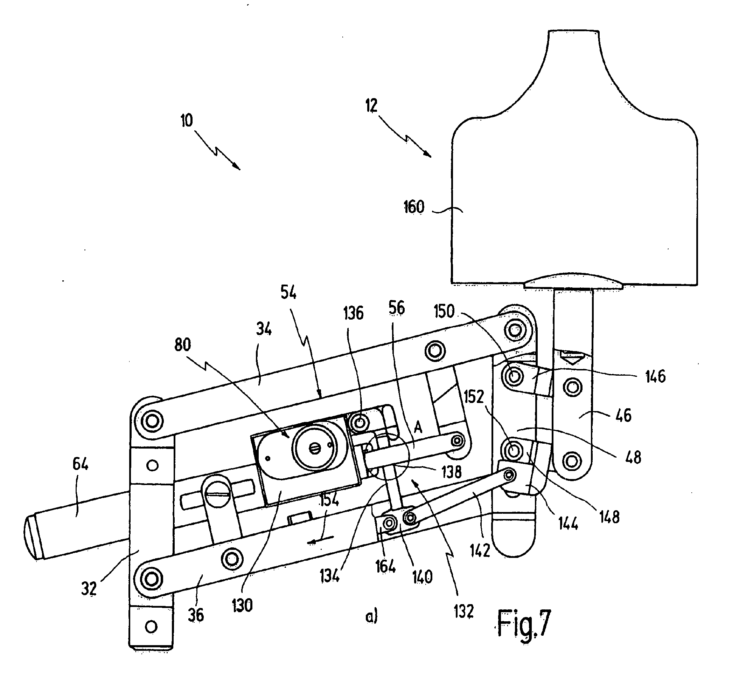

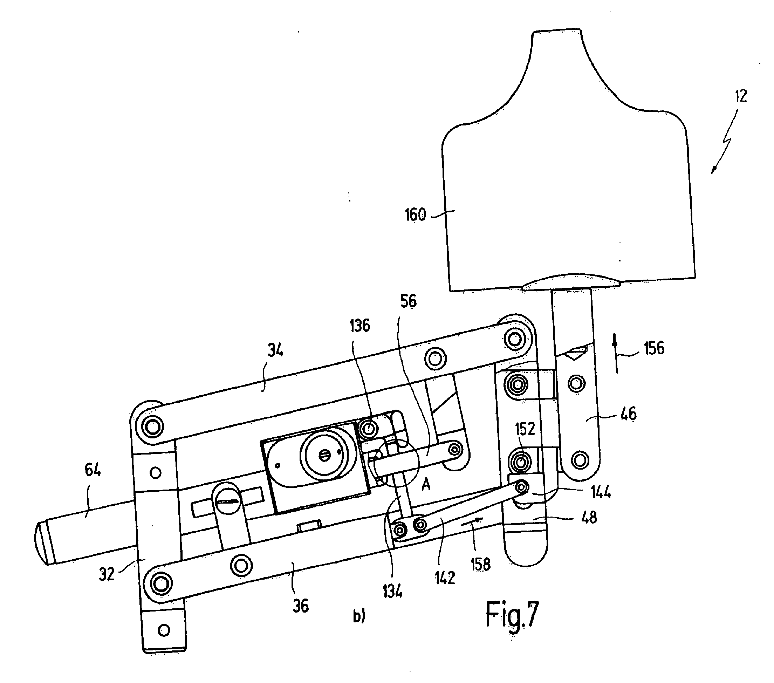

Damit die Armauflage 12 in der in Figur 6b) dargestellten Position sicher gegen ein unerwünschtes Absenken verriegelt ist, ist eine Raste 132 vorgesehen, die nachfolgend mit Bezug auf Figuren 7 und 8 näher beschrieben wird.

Die Raste 132 ist so ausgelegt, dass sie die längsgerichtete Bewegung der Stange 56 sperrt, wenn die Raste in ihrer Verriegelungsstellung ist, während die Raste gelöst werden kann, um die längsgerichtete Bewegung der Stange 56 freizugeben. In dem gezeigten Ausführungsbeispiel ist die Raste 132 so ausgestaltet, dass sie die Bewegung der Stange 56 relativ zu dem Zylinder 64 sperren kann.

Die Raste 132 weist einen Riegel 134 auf, der mit einem Ende an dem zylinderfesten Hauptgehäuse 130 um eine Kippachse 136 verschwenkbar angelenkt ist.

Der Riegel 134 weist eine Durchführung 138 auf, durch die die Stange 56 hindurchgeht. Je nach dem Kippzustand des Riegels 134 um die Kippachse 136 geht die Stange 56 senkrecht oder schräg durch die Durchführung 138 hindurch.

Figur 8a) zeigt die Raste 132 zusammen mit der Stange 56 in vergrößertem Maßstab in Alleinstellung. Die Stellung des Riegels 134 der Raste 132 in Figur 8a) entspricht dem Betriebszustand in Figur 7a). In Figur 8a) ist erkennbar, dass der Riegel 134 derart um die Kippachse 136 verkippt ist, dass die Stange 56 schräg durch die Durchführung 138 hindurchgeht, wodurch die Stange 56 in der Durchführung aufgrund von Verkantung mit dem Rand der Durchführung 138 geklemmt ist.

Figur 8b) zeigt die Anordnung aus Raste 132 und Stange 56 dagegen in einem Zustand, der dem Betriebszustand der Armstütze in Figur 7b) entspricht, in dem der Riegel 134 der Raste 132 derart um die Kippachse 136 verkippt ist, dass die Stange 56 nun senkrecht durch die Durchführung 138 hindurchgeht. In dieser Stellung der Raste 132 ist die Stange 56 in ihrer Längsrichtung beweglich.

Mit Bezug auf Figuren 7a) und b) wird nun beschrieben, wie die Raste 132 zum Sperren der Beweglichkeit der Stange 56 aktiviert und zum Entsperren gelöst wird.

Ein der Kippachse 136 gegenüberliegendes Ende 140 des Riegels 134 ist über ein Zug-/Schubelement 142, beispielsweise eine Stange oder Strebe, mit einem Hebelteil 144 verbunden, wobei die Verbindung des Zug-/Schubelements 142 mit dem Ende 140 des Riegels 134 und mit dem Hebelteil 144 gelenkig ist.

Die Armauflage 12 ist, wie bereits oben beschrieben, über das Stützelement 46 mit der Strebe 48 verbunden, die mit den Schwenkarmen 34 bis 40 verbunden ist. Die Verbindung der Stütze 46 mit der Strebe 48 ist jedoch nicht starr, sondern über zwei weitere Streben 146 und 148 bewerkstelligt, die mit der Strebe 48 gelenkig verbunden sind, so dass die Stütze 46 und damit die Armauflage 12 geringfügig relativ zu den Schwenkarmen 34 bis 40 beweglich ist. Diese Beweglichkeit ist entsprechend einer Anlenkung der Streben 146 und 148 über im Wesentlichen horizontale Schwenkachsen 150, 152 an der Strebe 148 im Wesentlichen vertikal gerichtet.

Das zuvor beschriebene Hebelteil 144 bildet einen L-Hebel, so dass eine Verschwenkung der Strebe 148 um die Schwenkachse 152 gleichzeitig eine Verschwenkung des Hebelteils 144 bewirkt, wodurch wiederum das Zug-/Schubelement 142 in seiner Längsrichtung bewegt wird.

In Figur 7a) ist ein Zustand dargestellt, in dem sich die Armauflage 12 und damit die Stütze 46 relativ zu den Schwenkarmen 34 bis 40 in der maximalen nach unten verschwenkten Stellung um die Schwenkachsen 150 bzw. 152 befindet. Diese Stellung nimmt die Armauflage 12 selbsttätig aufgrund ihrer Gewichtskraft ein und außerdem dann, wenn sich eine Bedienungsperson mit ihrem Arm auf der Armauflage 12 abstützt. In diesem Zustand drückt das Hebelteil 144 das Zug-/Schubelement in Richtung eines Pfeiles 154, wodurch der Riegel 134 der Raste 132, wie in Figur 8a) dargestellt, um die Kippachse 136 derart verkippt ist, dass die Stange 56 schräg durch die Durchführung 138 hindurchgeht und somit geklemmt ist. Die Klemmwirkung ist umso höher, je größer die auf die Armauflage 12 ausgeübte Stützkraft ist.

Um nun die Raste 132 zu lösen, so dass sich die Stange 56 relativ zu dem Zylinder 64 bewegen kann, beispielsweise um ausgehend von Figur 7a) die Armauflage 12 abzusenken, wird die Armauflage gemäß einem Pfeil 156 in Figur 7b) geringfügig angehoben, wobei dieses geringfügige Anheben keine aufwärts gerichtete Schwenkbewegung der Schwenkarme 34 bis 40 bewirkt, sondern lediglich eine Relativbewegung der Armauflage 12 zu den Schwenkarmen 34 bis 40. Bei dieser Relativbewegung zieht das Hebelteil 144 das Zug-/Schubelement 142 in Richtung eines Pfeiles 158, wodurch der Riegel 134 der Raste 132 um die Kippachse 136 so verkippt wird, dass die Stange 56 nunmehr senkrecht durch die Durchführung 138 hindurchgeht und sich nunmehr relativ zur Durchführung 138 bewegen kann. Da nunmehr die Beweglichkeit der Stange 56 freigegeben ist, kann die Armauflage 12 abgesenkt werden. Das Absenken erfolgt durch die Wirkung der Feder 66, wobei die Armauflage 12 dabei weiter in der gegenüber den Schwenkarmen 38 bis 40 geringfügig angehobenen Position (Figur 7b)) gehalten wird, um die Raste 132 entriegelt zu halten. Das Bremselement 100 gewährleistet dabei eine kontrollierte Absenkung mit einer der eingestellten Bremswirkung entsprechenden Geschwindigkeit.

Das Lösen der Raste 132 erfordert lediglich ein geringfügiges Anheben der Armauflage 12, was mit demselben Arm durchgeführt werden kann, der sich gerade in der Armauflage abstützt. Damit mit diesem Arm die Armauflage 12 nach oben angehoben werden kann, ist die Armauflage 12 in Form einer Schale 160 ausgebildet, die im Wesentlichen die Form eines C aufweist, so dass ein Abschnitt 162 zumindest bis zur Oberseite des in der Schale 160 aufgenommenen Arms reicht.

Wie aus Figuren 1, 6 und 7 hervorgeht, ist das untere Ende 140 des Riegels 134, das Zug-/Schubelement 142 und das Hebelteil 144 sowie die Streben 146 und 148 teilweise in dem Schwenkarm 40 bzw. der Strebe 48 versenkt aufgenommen.

Des Weiteren ist das untere Ende 140 des Riegels 134 über ein weiteres Zugelement 164 mit einer nicht dargestellten Zugfeder in dem Schwenkarm 40 verbunden, um zu bewirken, dass der Riegel 134 in die Verriegelungsstellung gemäß Figur 8a) vorgespannt ist.

## Patentansprüche

1. Chirurgische Armstütze, mit einer Armauflage (12) und einer Tragstruktur (14) für die Armauflage (12), wobei die Tragstruktur (14) eine Basis (16) und zumindest einen Schwenkarm (34, 38) aufweist, der einerseits an der Basis (16) um eine zumindest näherungsweise horizontale Schwenkachse (42) verschwenkbar angelenkt und andererseits mit der Armauflage (12) verbunden ist, so dass eine Verschwenkung des zumindest einen Schwenkarms (34, 38) um die Schwenkachse (42) eine Höhenverstellung der Armauflage (12) bewirkt, und wobei dem zumindest einen Schwenkarm (34, 38) ein Steuermechanismus (54) zugeordnet ist, der wahlweise ein Feststellen und Verschwenken des zumindest einen Schwenkarms (34, 38) in Abwärtsrichtung und in Aufwärtsrichtung ermöglicht, wobei der Steuermechanismus (54) zumindest eine Stange (56) aufweist, die beim Verschwenken des zumindest einen Schwenkarms (34, 38) zumindest in ihrer Längsrichtung beweglich ist, **dadurch gekennzeichnet, dass** die Stange (56) mit einer Freilaufkupplung (80) zusammenwirkt, die ein erstes Kupplungselement (82) und ein zweites Kupplungselement (84) aufweist, wobei das erste Kupplungselement (82) von der Stange (56) angetrieben wird und bei einer Aufwärtsbewegung des zumindest einen Schwenkarms (34, 38) gegenüber dem zweiten Kupplungselement (84) frei beweglich ist und bei einer Abwärtsbewegung des zumindest einen Schwenkarms (34, 38) das zweite Kupplungselement (84) mit antreibt, und dass das zweite Kupplungselement (84) mittels zumindest eines Bremselements (100) gebremst ist.

2. Armstütze nach Anspruch 1, **dadurch gekennzeichnet, dass** das erste Kupplungselement (82) und das zweite Kupplungselement (84) bei der Abwärtsbewegung des zumindest einen Schwenkarms (34, 38) über einen Formschluss miteinander in Verbindung stehen, während bei einer Aufwärtsbewegung des zumindest einen Schwenkarms (34, 38) der Formschluss durch eine Beabstandung des zweiten Kupplungselements (84) von dem ersten Kupplungselement (82) aufgehoben ist.

3. Armstütze nach Anspruch 1 oder 2, **dadurch gekennzeichnet, dass** das erste Kupplungselement (82) und das zweite Kupplungselement (84) um eine gemeinsame Drehachse (86) drehbar sind und auf ihren einander zugewandten Stirnseiten eine komplementäre Schrägverzahnung (88, 90) aufweisen, die in der einen Drehrichtung des ersten Kupplungselements (82) ein Abstoßen des zweiten Kupplungselements (84) und in der entgegengesetzten Richtung des ersten Kupplungselements (82) eine Drehbewegung des zweiten Kupplungselements (84) bewirkt.

4. Armstütze nach Anspruch 3, **dadurch gekennzeichnet, dass** das zweite Kupplungselement (84) in Richtung der Drehachse (86) axial beweglich und in Richtung zum ersten Kupplungselement (82) hin vorgespannt ist.

5. Armstütze nach Anspruch 3 oder 4, **dadurch gekennzeichnet, dass** die gemeinsame Drehachse (86) zumindest annähernd senkrecht zur axialen Bewegungsrichtung der Stange (56) verläuft.

6. Armstütze nach einem der Ansprüche 1 bis 5, **dadurch gekennzeichnet, dass** die Stange (56) das erste Kupplungselement (82) über eine Getriebeanordnung (120) antreibt.

7. Armstütze nach Anspruch 6, **dadurch gekennzeichnet, dass** die Getriebeanordnung (120) zumindest ein Zahnrad (122) aufweist, das mit einer Verzahnung (124) der Stange (56) kämmt.

8. Armstütze nach Anspruch 7, **dadurch gekennzeichnet, dass** die Getriebeanordnung (120) zumindest zwei miteinander kämmende Zahnräder (122, 126) aufweist, die vorzugsweise einen verschiedenen Durchmesser aufweisen.

9. Armstütze nach einem der Ansprüche 1 bis 8, **dadurch gekennzeichnet, dass** die durch das Bremselement (100) bereitgestellte Bremswirkung einstellbar ist.

10. Armstütze nach Anspruch 9, **dadurch gekennzeichnet, dass** das Bremselement (100) aus einem elastischen Material gefertigt ist, und dass eine Spanneinrichtung mit dem Bremselement (100) in Wirkverbindung steht, um den Anpressdruck des Bremselements (100) an das zweite Kupplungselement (84) zu variieren.

11. Armstütze nach einem der Ansprüche 1 bis 10, **dadurch gekennzeichnet, dass** der Stange (56) zumindest eine Feder (66) zugeordnet ist, die bei einer Aufwärtsbewegung des zumindest einen Schwenkarms (34, 38) gespannt und bei einer Abwärtsbewegung entspannt wird.

12. Armstütze nach Anspruch 11, **dadurch gekennzeichnet, dass** die zumindest eine Feder (66) so ausgelegt ist, dass der zumindest eine Schwenkarm (34, 38) durch die Kraft der Feder (66) gegen die von dem Bremselement (100) erzeugte Bremswirkung in Abwärtsrichtung verschwenkt wird.

13. Armstütze nach einem der Ansprüche 1 bis 12, **dadurch gekennzeichnet, dass** die Tragstruktur (14) zumindest einen zweiten Schwenkarm (36, 40) aufweist, der zumindest näherungsweise parallel zu dem ersten Schwenkarm (34, 38) verläuft und einerseits an der Basis (16) um eine von der Schwenkachse (42) des ersten Schwenkarmes (34, 38) vertikal beabstandete Schwenkachse (44) angelenkt und andererseits mit der Armauflage (12) verbunden ist, und dass die Stange (56) teilweise in einem Zylinder (64) relativ zu diesem beweglich aufgenommen ist, und wobei der Zylinder (64) an dem zweiten Schwenkarm (36, 40) befestigt ist.

14. Armstütze nach Anspruch 13, **dadurch gekennzeichnet, dass** die Freilaufkupplung (80) ein Gehäuse (130) aufweist, das an dem Zylinder (64) befestigt ist.

15. Armstütze nach einem der Ansprüche 1 bis 14, **dadurch gekennzeichnet, dass** für die Stange (56) eine lösbare Raste (132) vorgesehen ist, die die Stange (56) axial unbeweglich sperrt und nach Lösen freigibt.

16. Armstütze nach Anspruch 15, **dadurch gekennzeichnet, dass** die Raste (132) durch einen Riegel (134) gebildet wird, der eine Durchführung (138) für die Stange (56) aufweist, wobei der Riegel (134) um eine Kippachse (136) in eine erste Kippstellung, in der die Stange (56) schräg durch die Durchführung (138) hindurchgeht und dadurch an der Durchführung (138) geklemmt ist, und in eine zweite Kippstellung bewegbar ist, in der die Stange (56) im Wesentlichen senkrecht durch die Durchführung (138) hindurchgeht und dadurch axial beweglich ist.

17. Armstütze nach Anspruch 15 oder 16, **dadurch gekennzeichnet, dass** die Armauflage (12) relativ zu dem zumindest einen Schwenkarm (34, 38) beweglich ist, und dass die Raste (132) mit der Armauflage (12) derart verbunden ist, dass durch geringfügiges Anheben der Armauflage (12) relativ zum zumindest einen Schwenkarm (34 38) die Raste (132) aus ihrer Sperrstellung in die Freigabestellung und durch geringfügiges Absenken der Armauflage (12) relativ zu dem zumindest einen Schwenkarm (34, 38) in die Sperrstellung überführt wird.

18. Armstütze nach einem der Ansprüche 1 bis 17, **dadurch gekennzeichnet, dass** die Armauflage (12) so ausgebildet ist, dass sie ein Anheben der Armauflage (12) mit demselben Arm ermöglicht, der sich auf der Armauflage (12) abstützt.

19. Armstütze nach Anspruch 18, **dadurch gekennzeichnet, dass** die Armauflage (12) als im Wesentlichen C-förmige Schale (160) ausgebildet ist.

## Claims

1. A surgical armrest, comprising an arm support (12) and a carrying structure (14) for the arm support (12), the carrying structure (14) having a base (16) and at least one pivot arm (34, 38) which, on the one hand, is articulated on the base (16) so as to pivot about an at least approximately horizontal pivot axis (42), and, on the other hand, is connected to the arm support (12), such that a pivoting of the at least one pivot arm (34, 38) about the pivot axis (42) causes a height adjustment of the arm support (12), and the at least one pivot arm (34, 38) being assigned a control mechanism (54) which selectively permits a fixing and a pivoting of the at least one pivot arm (34, 38) in the downward direction and in the upward direction, the control mechanism (54) having at least one bar (56) which is movable, at least in its longitudinal direction, upon pivoting of the at least one pivot arm (34, 38), **characterized in that** the bar (56) interacts with a freewheel coupling (80) having a first coupling element (82) and a second coupling element (84), wherein the first coupling element (82) is driven by the bar (56) and is freely movable relative to the second coupling element (84) upon an upward movement of the at least one pivot arm (34, 38), and, upon a downward movement of the at least one pivot arm (34, 38), drives the second coupling element (84), and **in that** the second coupling element (84) is braked by means of at least one brake element (100).

2. The armrest of Claim 1, **characterized in that** the first coupling element (82) and the second coupling element (84) are in connection with one another via a form-fit engagement during the downward movement of the at least one pivot arm (34, 38), whereas, during an upward movement of the at least one pivot arm (34, 38), the form-fit engagement is cancelled by the second coupling element (84) being spaced away from the first coupling element (82).

3. The armrest of Claim 1 or 2, **characterized in that** the first coupling element (82) and the second coupling element (84) are rotatable about a common rotation axis (86) and, on their end faces directed toward one another, have a complementary oblique toothing (88, 90) which, in one direction of rotation of the first coupling element (82), causes a pushing-away of the second coupling element (84) and, in the opposite direction of the first coupling element (82), causes a rotational movement of the second coupling element (84).

4. The armrest of Claim 3, **characterized in that** the second coupling element (84) is axially movable in the direction of the rotation axis (86) and is pretensioned in the direction toward the first coupling element (82).

5. The armrest of Claim 3 or 4, **characterized in that** the common rotation axis (86) extends at least approximately perpendicular to the axial direction of movement of the bar (56).

6. The armrest of any one of Claims 1 through 5, **characterized in that** the bar (56) drives the first coupling element (82) via a gear arrangement (120).

7. The armrest of Claim 6, **characterized in that** the gear arrangement (120) has at least one toothed wheel (122) that meshes with a toothing (124) of the bar (56).

8. The armrest of Claim 7, **characterized in that** the gear arrangement (120) comprises at least two toothed wheels (122, 126) that mesh with one another and preferably have a different diameter.

9. The armrest of any one of Claims 1 through 8, **characterized in that** the braking action brought about by the brake element (100) is adjustable.

10. The armrest of Claim 9, **characterized in that** the brake element (100) is made from an elastic material, and **in that** a clamping device is operatively connected to the brake element (100) in order to vary the contact pressure of the brake element (100) on the second coupling element (84).

11. The armrest of any one of Claims 1 through 10, **characterized in that** the bar (56) is assigned at least one spring (66) which is tensioned during an upward movement of the at least one pivot arm (34, 38) and is relaxed during a downward movement.

12. The armrest of Claim 11, **characterized in that** the at least one spring (66) is configured such that the at least one pivot arm (34, 38) is pivoted in the downward direction by the force of the spring (66) counter to the braking action generated by the brake element (100).

13. The armrest of any one of Claims 1 through 12, **characterized in that** the carrying structure (14) comprises at least one second pivot arm (36, 40) which extends at least approximately parallel to the first pivot arm (34, 38) and at one end is articulated on the base (16) about a pivot axis (44) spaced vertically apart from the pivot axis (42) of the first pivot arm (34, 38) and at the other end is connected to the arm support (12), and **in that** the bar (56) is partially received in a cylinder (64) so as to be movable relative to the latter, the cylinder (64) being secured on the second pivot arm (36, 40).

14. The armrest of Claim 13, **characterized in that** the freewheel coupling (80) comprises a housing (130) which is secured on the cylinder (64).

15. The armrest of any one of Claims 1 through 14, **characterized in that** the bar (56) is provided with a releasable catch (132) which blocks the bar (56) so that it is axially immovable and frees it after release.

16. The armrest of Claim 15, **characterized in that** the catch (132) is formed by a bolt (134) which has a passage (138) for the bar (56), the bolt (134) being able to be moved about a tilt axis (136) into a first tilt position, in which the bar (56) extends obliquely through the passage (138) and is thus clamped on the passage (138), and into a second tilt position in which the bar (56) extends substantially perpendicularly through the passage (138) and is thus axially movable.

17. The armrest of Claim 15 or 16, **characterized in that** the arm support (12) is movable relative to the at least one pivot arm (34, 38), and **in that** the catch (132) is connected to the arm support (12) in such a way that, by slight lifting of the arm support (12) relative to the at least one pivot arm (34, 38), the catch (132) is transferred from its blocked position into the release position, and, by slight lowering of the arm support (12) relative to the at least one pivot arm (34, 38), is transferred into the blocked position.

18. The armrest of any one of Claims 1 through 17, **characterized in that** the arm support (12) is designed in such a way that it allows the arm support (12) to be lifted by the same arm that is resting on the arm support (12).

19. The armrest of Claim 18, **characterized in that** the arm support (12) is designed as a substantially C-shaped shell (160).

## Revendications

1. Accoudoir chirurgical, comportant un repose-bras (12) et une structure de support (14) pour le repose-bras (12), la structure de support (14) présentant une base (16) et au moins un bras articulé (34, 38) qui est d'une part articulé à la base (16) autour d'un axe d'articulation horizontal (42) au moins dans une situation de rapprochement et d'autre part, est relié au repose-bras (12) de sorte qu'un pivotement d'au moins un bras articulé (34, 38) autour de l'axe d'articulation (42) entraîne un réglage en hauteur du repose-bras (12) et un mécanisme de commande (54) étant affecté à au moins un bras articulé (34, 38) qui permet à volonté une fixation et un pivotement d'au moins un bras d'articulation (34, 38) vers le bas et vers le haut, le mécanisme de commande (54) présentant au moins une tige (56) qui est mobile lors du pivotement d'au moins un bras d'articulation (34, 38) au moins dans sa direction longitudinale, **caractérisé en ce que** la tige (56) coopère avec un couplage à roue libre (80) qui présente un premier élément de couplage (82) et un deuxième élément de couplage (84), le premier élément de couplage (82) étant actionné par la tige (56) et étant librement mobile dans un mouvement vers le haut d'au moins un bras d'articulation (34, 38) par rapport au deuxième élément de couplage (84) et actionnant en même temps dans un mouvement vers le bas d'au moins un bras d'articulation (34, 38) le deuxième élément de couplage (84) et **en ce que** le deuxième élément de couplage (84) est freiné par au moins un élément de freinage (100).

2. Accoudoir selon la revendication 1, **caractérisé en ce que** le premier élément de couplage (82) et ie deuxième élément de couplage (84) sont reliés l'un à l'autre lors du mouvement vers le bas d'au moins un bras d'articulation (34, 38) par un accouplement mécanique, alors que lors d'un mouvement vers le haut d'au moins un bras d'articulation (34, 38), l'accouplement mécanique est annulé par une séparation du deuxième élément de couplage (84) du premier élément de couplage (82).

3. Accoudoir selon la revendication 1 ou 2, **caractérisé en ce que** le premier élément de couplage (82) et le deuxième élément de couplage (84) peuvent tourner autour d'un axe de rotation commun (86) et présentent sur leurs côtés frontaux tournés l'un vers l'autre, une indentation perpendiculaire complémentaire (88, 90) qui entraîne dans une direction de rotation du premier élément de couplage (82), un rejet du deuxième élément de couplage (84) et dans la direction opposée du premier élément de couplage (82), un mouvement de rotation du deuxième élément de couplage (84).

4. Accoudoir selon la revendication 3, **caractérisé en ce que** le deuxième élément de couplage (84) est mobile axialement en direction de l'axe de rotation (86) et est précontraint dans la direction du premier élément de couplage (82).

5. Accoudoir selon la revendication 3 ou 4, **caractérisé en ce que** l'axe de rotation commun (86) évolue au moins perpendiculairement à la direction de mouvement axiale de la tige (56).

6. Accoudoir selon l'une des revendications 1 à 5, **caractérisé en ce que** la tige (56) du premier élément de couplage (82) est actionnée par un dispositif d'engrenage (120).

7. Accoudoir selon la revendication 6, **caractérisé en ce que** le dispositif d'engrenage (120) présente au moins une roue dentée (122) qui s'engrène avec une indentation (124) de la tige (56).

8. Accoudoir selon la revendication 7, **caractérisé en ce que** le dispositif d'engrenage (120) présente au moins deux roues dentées (122, 126) s'engrenant l'une dans l'autre, qui présentent de préférence un diamètre différent.

9. Accoudoir selon l'une des revendications 1 à 8, **caractérisé en ce que** l'action de freinage assurée par l'élément de freinage (100) est réglable.

10. Accoudoir selon la revendication 9, **caractérisé en ce que** l'élément de freinage (100) est en un matériau élastique et **en ce qu'**un dispositif de tension est en relation active avec l'élément de freinage (100) pour faire varier la pression d'appui de l'élément de freinage (100) sur le deuxième élément de couplage (84).

11. Accoudoir selon l'une des revendications 1 à 10, **caractérisé en ce qu'**au moins un ressort (66) est affecté à la tige (56), qui est tendu par un mouvement vers le haut d'au moins un bras d'articulation (34, 38) et est relâché par un mouvement vers le bas.

12. Accoudoir selon la revendication 11, **caractérisé en ce qu'**au moins un ressort (66) est configuré de telle sorte qu'au moins un bras d'articulation (34, 38) pivote par la force du ressort (66) à l'encontre de l'action de freinage générée par l'élément de freinage (100) dans la direction vers le bas.

13. Accoudoir selon l'une des revendications 1 à 12, **caractérisé en ce que** la structure de support (14) présente au moins un deuxième bras d'articulation (36, 40) qui évolue au moins parallèlement, dans une situation de rapprochement, au premier bras d'articulation (34, 38) et est d'une part articulé à la base (16) autour d'un axe d'articulation (44) distant verticalement de l'axe d'articulation du premier bras d'articulation (34, 38) et d'autre part, est relié au repose-bras (12), et **en ce que** la tige (56) est reçue partiellement dans un cylindre (64) de façon mobile par rapport à celui-ci et le cylindre (64) étant fixé au deuxième bras d'articulation (36, 40).

14. Accoudoir selon la revendication 13, **caractérisé en ce que** le couplage à roue libre (80) présente un boîtier (130) qui est fixé au cylindre (64).

15. Accoudoir selon l'une des revendications 1 à 14, **caractérisé en ce que**, pour la tige (56), un arrêt détachable (132) est prévu, qui bloque la tige (56) de façon immobile axialement et la libère après son détachement.

16. Accoudoir selon la revendication 15, **caractérisé en ce que** l'arrêt (132) est formé par un penne (134) qui présente un passage (138) pour la tige (56), le penne (134) étant mobile autour d'un axe d'inclinaison (136) dans une première position d'inclinaison dans laquelle la tige (56) passe perpendiculairement dans le passage (138) et est ainsi bloquée dans le passage (138) et dans une deuxième position d'inclinaison dans laquelle la tige (56) traverse substantiellement perpendiculairement le passage (138) et est ainsi mobile axialement.

17. Accoudoir selon la revendication 15 ou 16, **caractérisé en ce que** le repose-bras (12) est mobile par rapport à au moins un bras d'articulation (34, 38) et **en ce que** l'arrêt (132) est relié au repose-bras (12) de telle sorte que par un léger soulèvement du repose-bras (12) par rapport à au moins un bras d'articulation (34, 38), l'arrêt (132) passe de sa position de blocage en position de déblocage et par un léger abaissement du repose-bras (12) par rapport à au moins un bras d'articulation (34, 38), passe à la position de blocage.

18. Accoudoir selon l'une des revendications 1 à 17, **caractérisé en ce que** le repose-bras (12) est conçu de telle sorte qu'il permette un soulèvement du repose-bras (12) avec le même bras que celui appuyant sur le repose-bras (12).

19. Accoudoir selon la revendication 18, **caractérisé en ce que** le repose-bras (12) est conçu essentiellement en forme de coque en C (160).
